# EUROPÄISCHE PATENTSCHRIFT

(11) **EP 2 415 414 B1**
(45) Veröffentlichungstag und Bekanntmachung des Hinweises auf die Patenterteilung: **04.03.2020**
(21) Anmeldenummer: 11176610.1
(22) Anmeldetag: 04.08.2011
(51) Int. Cl.: A61B 18/02, A61B 18/00, A61B 90/00, A61B 50/30

(54) **Handgriff für ein Chirurgieinstrument, insbesondere Kryochirurgieinstrument**
Handle for a surgical instrument, in particular cryosurgery instrument
Poignée pour un instrument chirurgical, notamment un instrument de cryochirurgie

(30) Priorität: 04.08.2010 DE 102010036829
(43) Veröffentlichungstag der Anmeldung: 08.02.2012
(73) Patentinhaber: Erbe Elektromedizin GmbH, 72072 Tübingen (DE)
(72) Erfinder: Sick, Christian, 72141 Nehren (DE); Amann, Markus, 72070 Tübingen (DE); Besch, Hansjörg, 72810 Gomaringen (DE)
(74) Vertreter: Rüger Abel Patentanwälte PartGmbB

(56) Entgegenhaltungen:
- EP-A2- 0 191 297
- WO-A1-99/65410
- WO-A2-2005/000106
- DE-T2- 60 026 041

## Beschreibung

Die Erfindung betrifft einen Handgriff für ein Chirurgieinstrument, insbesondere Kryochirurgieinstrument, mit einem Griffelement und wenigstens einem Hüllschlauch, der in einem Anschlussbereich in das Griffelement eingeschoben und fluiddicht mit diesem verbunden ist.

Über einen solchen Hüllschlauch werden bei den bekannten Handgriffen Zu- bzw. Ableitungsschläuche zum Griffelement geführt und dort befestigt, um beispielsweise eine Fluidversorgung für eine Sonde, die an den Handgriff anschließbar ist, zu ermöglichen. Solche Handgriffe für Chirurgieinstrumente sind also aus dem Stand der Technik bekannt.

Die Sterilität ist beim Einsatz von Chirurgieinstrumenten ein Hauptaugenmerk, um ein sicheres Eingreifen in den menschlichen Körper zu garantieren und die Abheilung der Wunden nach operativen Eingriffen nicht unnötig zu gefährden oder zu verlängern. Insofern besteht ein hoher Bedarf an chirurgischen Geräten und insbesondere an Handgriffen für Chirurgieinstrumente, die nach dem Gebrauch einer maschinellen Reinigung zugeführt werden können und insbesondere in geeigneten Waschmaschinen aufbereitbar sind.

Viele aus dem Stand der Technik bekannte Sonden, Handgriffe und dergleichen Chirurgiegeräte sind per se nicht waschmaschinentauglich, sodass eine Abdichtung zwischen Handgriff und Schlauchsatz folglich nicht vorgesehen war. Hier wurden meist Einweg-Teile verwendet, was eine große Abfallbelastung für die Umwelt darstellte und einen hohen Materialaufwand sowie hohe Kosten mit sich brachte.

Bei manchen flexiblen chirurgischen Sonden wurde bisher auf eine mechanische Verklemmung des Hüllschlauchs mit dem Griffelement durch eine Schraubenverbindung zurückgegriffen. Über die Schraubverbindung wurde der Hüllschlauch zudem am Griffelement angedichtet. Ein zusätzlicher Einbau einer Druckfeder als Kompensationselement sollte dabei einem Setzverhalten aufgrund einer thermischen Aufbereitung entgegen wirken. Dies garantierte jedoch auf Dauer keine verlässliche Abdichtung, da es aufgrund der thermischen Belastungen trotzdem zu Undichtigkeiten kam.

Auch ist es aus dem Stand der Technik bekannt den Hüllschlauch mit Silikon gegen das Griffelement abzudichten und insbesondere zu verkleben.

Ein weiteres Verfahren ist dadurch gekennzeichnet, das Griffelement gegen den Hüllschlauch anzuspritzen. Hier kam es jedoch insbesondere aufgrund einer Ovalisierung des Schlauchs beim Abknicken zu einer Spaltbildung zwischen dem Hüllschlauch und dem Schlauch und so zu einem Unterwandern der Tülle mit Flüssigkeit oder Partikeln beim Waschvorgang.

In der DE 600 26 041 T2 ist eine Schutzhülle offenbart, die zur Verwendung bei einer kryochirurgischen Sonde in einem Joule-Thomson-System mit einem geschlossenen Kreislauf geeignet ist. Ein geschlossener Kreislauf bedeutet, dass das unter Druck stehende Gas nach der Expansion in Zirkulation versetzt und nicht an die Atmosphäre abgegeben wird. Diese Systeme sind in der Regel dauerhaft abgedichtet, um eine Einleitung von Verunreinigungen zu verhindern. Die aus dieser Druckschrift bekannte Schutzhülle wird als elastisches Bauteil so auf die Sonde aufgeschoben, dass sie diese gegen die Umgebung abdichtet. Durch die so erzielte sterile Barriere der einzelnen Komponenten kann die Sonde in einem chirurgischen Eingriff verwendet werden. Diese Hülle gewährleistet die Wärmeleitung der Kryosondenspitze mittels einer angegossenen Metallkappe. Der restliche Teil der Hülle besteht aus einem thermisch nicht leitenden Material, wie beispielsweise Kunststoff, und stellt somit eine Wärmeisolierung dar. Die Schutzhülle muss jedoch für jede Art von Kryosonde dem exakten Aufbau angepasst werden, um einen sicheren Halt zu ermöglichen. Dies ist sehr aufwendig. Auch das Aufbringen der Schutzhülle kann sich schwierig gestalten, da sie nicht beschädigt werden darf, um ihren oben genannten Zweck der sterilen Abdeckung der Komponenten zu erfüllen.

Der Erfindung liegt daher die Aufgabe zugrunde, einen Handgriff für ein chirurgisches Instrument, insbesondere Kryochirurgieinstrument, der eingangs genannten Art zu schaffen, bei dem eine Reinigung und insbesondere eine maschinelle Reinigung mittels einer Waschmaschine, ohne Beschädigung des Handgriffes möglich ist. Dabei soll der Schutz vor Flüssigkeit und Partikeln sowie eine komfortable und sichere Handhabung des Handgriffs für den Chirurgen gewährleistet werden.

Diese Aufgabe wird durch einen Handgriff nach Patentanspruch 1 gelöst.

Insbesondere wird die Aufgabe durch einen Handgriff für ein Chirurgieinstrument, insbesondere Kryochirurgieinstrument, gelöst, mit einem Griffelement und wenigstens einem Hüllschlauch, der in einem Anschlussbereich in das Griffelement eingeschoben und fluiddicht mit diesem verbunden ist, wobei im Innenraum des Hüllschlauches wenigstens ein Stützelement derart ausgebildet und angeordnet ist, dass der Hüllschlauch im Anschlussbereich zwischen dem Griffelement und dem Stützelement eingeklemmt ist.

Wie erwähnt, werden über einen solchen Hüllschlauch Zu- bzw. Ableitungselemente zum Griffelement geführt und dort befestigt, um beispielsweise eine Fluidversorgung für eine Sonde, die an den Handgriff anschließbar ist, zu ermöglichen. Diese Zu- und Ableitungselemente sind beispielsweise solche Schläuche, die benötigt werden, um das für den jeweiligen chirurgischen Eingriff nötige Medium zur Sonde und wieder zur Quelle zurück zu führen.

Ein wesentlicher Punkt der Erfindung liegt darin, dass ein Handgriff für ein chirurgisches Instrument derart ausgebildet ist, dass er einen dichtenden Übergang zwischen Griffelement und Hüllschlauch aufweist. Diese Abdichtung wird dadurch ausgebildet, dass der Hüllschlauch innenseitig vom Griffelement gestützt wird und mit diesem "gestützten" Ende fluiddicht am Griffelement eingeklemmt wird bzw. ist. Auch bei thermischen Belastungen, wie sie beispielsweise in Waschmaschinen auftreten, werden so Setzungsspalte zwischen Griffelement und Hüllschlauch verhindert. Eine Spaltbildung zwischen Hüllschlauch und Griffelement wird demgemäß vermieden und ein Unterwandern des Griffelements mit Flüssigkeit oder Partikeln verhindert. Der Handgriff ist aufgrund dieser Abdichtung waschmaschinentauglich; seine Sterilität kann gewährleistet werden.

Dabei ist zu erwähnen, dass der erfindungsgemäße Handgriff für ein chirurgisches Instrument in jeglicher Art von gerätegestützter Chirurgie einsetzbar ist, wie beispielsweise in der Kryo-Chirurgie, Hochfrequenz(HF)-Chirurgie, Wasserstrahl-Chirugie, Ophthalmo-Chirurgie etc.

Durch das Einschieben bzw. Einlagern eines Stützelements im Innenraum des Hüllschlauches wird der dichtende Übergang vom Griffelement auf den Hüllschlauch abgestützt. Der Hüllschlauch wird durch das Stützelement gegen das Griffelement gedrückt bzw. gepresst und so sicher dazwischen eingeklemmt. Einem Abknicken des Hüllschlauchs wird somit entgegengewirkt und die Dichtwirkung der Verbindung von Hüllschlauch und Griffelement verstärkt.

Vorzugsweise ist der Anschlussbereich am proximalen Ende des Griffelements ausgebildet. Der Übergang vom Griffelement zum Hüllschlauch wird durch das Stützelement im Anschlussbereich optimal stabilisiert und wirkt dadurch einem Abknicken des Hüllschlauchs am proximalen Ende des Griffelementes entgegen.

Vorzugsweise ist das Griffelement im Anschlussbereich wenigstens teilweise aus einem elastischen Endelement ausgebildet bzw. weist das Griffelement im Anschlussbereich ein elastisch ausgebildetes Endelement auf. Eine solche Ausführungsform garantiert, dass sich das Griffelement infolge der elastischen Ausführung im Anschlussbereich und insbesondere am Übergang zum Hüllschlauch stets dichtend an den Hüllschlauch anpasst und somit sicher mit diesem verbunden ist bzw. bleibt.

Der restliche Teil des Griffelements, also insbesondere im distalen Bereich, ist vorzugsweise aus einem Polymer ausgebildet, das härter und insbesondere steifer ist als das elastische Endelement des Griffelements, wie beispielsweise Polyamid (PA) oder Polypropylen (PP). Aufgrund der resultierenden Griffelementhaptik kann dem Benutzer ein sicherer Umgang mit dem chirurgischen Instrument ermöglicht werden.

Das elastische Endelement des Griffelements ist vorzugsweise aus einem thermoplastischen Elastomer ausgebildet, das sich insbesondere bei einer Temperaturerhöhung zusammenzieht. Dadurch kann diese Anpassung an den Hüllschlauch optimiert und die Dichtwirkung verstärkt werden, da das thermoplastische Material bei einer thermischen Aufbereitung nachschrumpft. Weiterhin ist auch Silikon als elastisches Endelement verwendbar.

Vorzugsweise ist das elastische Endelement des Griffelements um den Hüllschlauch angespritzt oder dergleichen befestigt. Durch diese Befestigung kann die Dichtwirkung dauerhaft gewährleistet werden, da sich so das elastische Endelement im Anschlussbereich direkt an den Hüllschlauch und das Griffelement "anschmiegt". Ein Unterwandern des Griffelementes mit einer Flüssigkeit oder Partikeln kann verhindert und ein optimaler dichtender Übergang ausgebildet werden.

In bevorzugter Weise ist das Stützelement als eine Hülse ausgebildet. Diese Hülse wird in den Hüllschlauch eingeschoben, dehnt diesen etwas auf und stützt ihn so optimal ab. Der Hüllschlauch kann insbesondere seine Form im Anschlussbereich nicht mehr so verändern, dass es zu einem Abknicken des Hüllschlauchs in diesem Bereich kommt. Insofern wird in entscheidendem Maße die Dichtwirkung verstärkt.

Das Stützelement steht mit einem proximalen Ende aus dem Anschlussbereich des Griffelements hervor. Dies bewirkt, dass der Hüllschlauch über den Anschlussbereich und das elastische Endelement des Griffelements hinaus gestützt wird, wodurch der dichtende Übergang des Handgriffs zum Hüllschlauch des Schlauchsatzes stabilisiert wird. Insbesondere wird einer Spaltbildung durch eine Ovalisierung des Hüllschlauchs beim Abknicken verhindert. In einer bevorzugten Weise steht das Stützelement um eine Länge, die im Wesentlichen dem Durchmesser des Hüllschlauches entspricht über, insbesondere um eine Länge zwischen 1 mm bis 10 mm, insbesondere 5 mm.

Vorzugsweise weist der Handgriff wenigstens ein Klemmelement auf, das den Hüllschlauch insbesondere mit einem distalen Ende auf dem Stützelement einklemmt. Das über das Stützelement gestülpte distale Ende des Hüllschlauchs wird mit einem zusätzlichen Klemmelement am Stützelement befestigt und gesichert und so im Griffelement gelagert. Insbesondere kann so ein fester Halt des Hüllschlauchs im Griffelement gewährleistet werden.

In bevorzugter Weise ist das Klemmelement als ein Klemmring ausgebildet, der über den Hüllschlauch in Richtung einer Klemmfläche am Stützelement derart aufschiebbar ist, dass der Hüllschlauch zwischen Klemmfläche und Klemmring eingeklemmt ist. An die insbesondere leicht abgeschrägte Klemmfläche des Stützelements wird der Hüllschlauch derart angepresst, dass der Klemmring, der eine vorzugsweise in die entgegen gesetzte Richtung abgeschrägte komplementäre Fläche aufweist, sich an die abgeschrägte Klemmfläche des Stützelements anpasst. Dies optimiert den Halt bzw. den festen und sicheren Sitz des Hüllschlauches auf dem Stützelement und sichert den Hüllschlauch so gegen ein Abrutschen bzw. Abgleiten vom Stützelement ab. Vorzugsweise ist wenigstens ein Dichtelement, insbesondere ein 0-Ring, dichtend zwischen dem Griffelement und dem Hüllschlauch und/oder zwischen dem Hüllschlauch und dem Stützelement angeordnet. Dieses Dichtelement wird beispielsweise in Axialrichtung des Hüllschlauches hinter dem Klemmring um den Hüllschlauch in einer Ausnehmung des Hartelements des Griffelements angeordnet. Es ergibt sich so ein weiteres Dichtungselement im Handgriff des chirurgischen Instruments, um ein Eindringen von einer Flüssigkeit oder Partikeln in das Innere des chirurgischen Instruments zu verhindern. Zudem verbessert dieses zusätzliche Dichtungselement den Sitz des Hüllschlauches auf dem Stützelement bzw. innerhalb des Griffelementes, so dass es zur Lagesicherung des Hüllschlauches beiträgt.

Weitere Ausführungsformen der Erfindung ergeben sich aus den Unteransprüchen.

Nachfolgend wird die Erfindung anhand von Ausführungsbeispielen beschrieben, die anhand der Abbildungen näher erläutert werden. Hierbei zeigen:
Fig. 1 : eine schematische Darstellung eines chirurgischen Instruments mit einer Versorgungseinheit;
Fig. 2 : einen Längsschnitt durch einen Handgriff des chirurgischen Instruments gemäß Fig.1;
Fig. 3 : einen Querschnitt durch den Handgriff nach Fig. 2 ;
Fig. 4 : einen Querschnitt durch den Handgriff nach Fig. 2 ; und
Fig. 5 : eine Detailansicht des in Fig. 2 gekennzeichneten Arretierungsbereiches.

In der nachfolgenden Beschreibung werden für gleiche und gleich wirkende Teile dieselben Bezugsziffern verwendet, wobei zur Unterscheidung bisweilen Hochindizes ihre Anwendung finden.

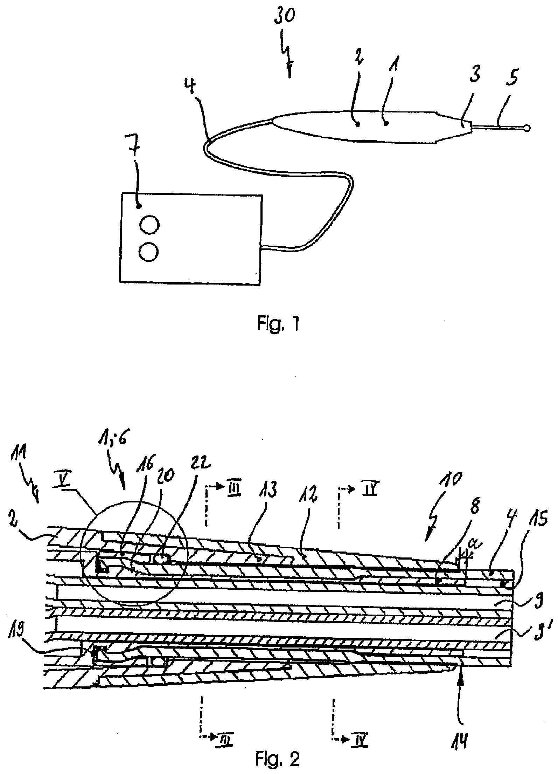

Fig. 1 zeigt eine schematische Darstellung eines chirurgischen Instruments 30 mit einer Versorgungseinheit 7. Das chirurgische Instrument 30 besteht aus einem Handgriff 1 mit einem Griffelement 2, und einem Sondenkopf 3 mit einer Sondenspitze 5. Es ist über den Hüllschlauch 4, der die Schläuche 9 für einen Zulauf und einen Rücklauf führt (siehe Fig. 2), mit der Versorgungseinheit 7 verbunden. Die Versorgungseinheit 7 stellt hier die Quelle für jegliche Versorgung bzw. Versorgungsmedien dar, je nachdem in welchem Gebiet das chirurgische Instrument eingesetzt wird. Solche Versorgungsmedien sind beispielsweise Wechselstrom für die Hochfrequenz(HF)-Chirurgie, Kühlmittel, vorzugsweise flüssiger Stickstoff, für die Kryochirurgie oder Wasser für die Wasserstrahl-Chirurgie etc.

In Fig. 2 ist ein Längsschnitt durch den Handgriff 1 des chirurgischen Instruments 30 gemäß Fig. 1 im Anschlussbereich 6 dargestellt, also dem Bereich an dem der Hüllschlauch 4 mit dem Griffelement 2 verbunden ist. Wie eingangs erwähnt, sind in den Handgriff 1 und insbesondere in den Hüllschlauch 4 zwei Schläuche 9, insbesondere für einen Zulauf und einen Rücklauf, eingeführt. Sie leiten das zum chirurgischen Eingriff benötigte Medium zum chirurgischen Instrument 30 und wieder von diesem weg.

Der Anschlussbereich 6 befindet sich am proximalen Ende 10 des Griffelements 2. Über ein Stützelement, hier eine Hülse 8, die im Innenraum 15 des Hüllschlauches 4 angeordnet und insbesondere über den Hüllschlauch 4 unter Aufweitung übergestülpt ist, wird der Hüllschlauch 4 gegen das Griffelement 2 verklemmt. Die Hülse B dient erfindungsgemäß zur sicheren Abstützung des Hüllschlauchs 4 im Anschlussbereich 6 und insbesondere dem Schutz gegen eine Ovalisierung oder Abknicken am proximalen Ende 10.

An der Hülse 8 befindet sich eine vorzugsweise radial umlaufende Klemmfläche 20, an der der Hüllschlauch 4 anliegt. Um die Befestigung des Hüllschlauchs 4 an der Hülse 8 zu optimieren, kann über den Hüllschlauch 4 ein Klemmelement, insbesondere ein Klemmring 16, übergeschoben werden. Zusätzlich wird der Hüllschlauch 4 zur Lagesicherung in Axialrichtung bei dieser Ausführungsform in einer Ausnehmung 19 verhakt.

Zur zusätzlichen Abdichtung des Handgriffs 1 ist in dieser Ausführungsform in Axialrichtung hinter dem Klemmring 16 ein Dichtelement, insbesondere ein O-Ring 22, eingefügt.

Am distalen Ende 11 des Griffelements 2 ist ein Hartelement 13 ausgebildet. Im Verbindungsbereich zwischen Griffelement 2 und Hüllschlauch 4 ist im Anschlussbereich 6 ein elastisches Endelement 12 des Griffelements 2 aufgebracht. Bei dieser Ausführungsform ist das elastische Endelement 12 auf den Hüllschlauch 4 und das Hartelement 13 aufgespritzt. Das elastische Endelement 12 bildet so eine elastische, den Bewegungen des Hüllschlauches bedingt folgende Schutzhülle, die den dichtenden Übergang zwischen Griffelement 2 und Hüllschlauch 4 sicherstellt und insbesondere das Eindringen von Flüssigkeit oder Partikeln aus der Umgebung verhindert.

Diese Dichtwirkung wird durch die Hülse 8 verstärkt, da diese den Hüllschlauch 4 noch zusätzlich aufdehnt und ihn so gegen das elastische Endelement 12 presst. Das chirurgische Instrument 30 ist dadurch waschmaschinentauglich und kann in einer Reinigungsmaschine optimal gereinigt und sterilisiert werden, um danach erneut in einem chirurgischen Eingriff eingesetzt zu werden.

Das elastische Endelement 12 ist aus einem Thermoplast ausgebildet, dass sich bei zyklischen Temperaturerschwankungen, also insbesondere bei aufeinanderfolgendem Erhitzen und anschließendem Abkühlen zusammenzieht. Durch die erhöhte Temperatur während des Reinigungsvorgangs und dem anschließenden Abkühlen zieht sich folglich das elastische Endelement 12 aufgrund seiner thermoplastischen Eigenschaften zusammen und verstärkt die Dichtwirkung.

Die Hülse 8 steht mit ihrem proximalen Ende 14 im Anschlussbereich 6 über das proximale Ende 10 des Griffelements 2 um eine Länge a hervor. Diese Länge a beträgt beispielsweise zwischen 1 mm und 10 mm, hier insbesondere 2 mm. Dadurch wird der Hüllschlauch 4 verstärkt abgestützt und Insbesondere ein Abknicken und eine daraus resultierende Ovalisierung verbunden mit einer Spaltbildung verhindert. Das elastische Endelement 12 des Griffelements 2 kann somit nicht von Flüssigkeit oder Partikeln unterwandert werden.

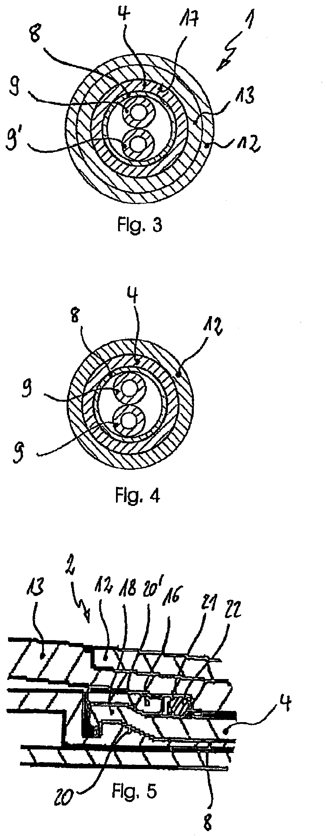

Fig. 3 zeigt einen Querschnitt durch den Handgriff gemäß Fig. 1 entlang der Schnittlinie aus Fig. 2 in dessen Anschlussbereich 6. Im Inneren des chirurgischen Instruments 30 sind die beiden Schläuche 9 zu sehen, die einen Zulauf und einen Rücklauf darstellen. Diese beiden Schläuche 9 sind durch die Hülse 8 in den Handgriff 1 des chirurgischen Instruments 30 geführt. Die Hülse 8 stützt den Hüllschlauch 4 derart ab, dass dieser sicher und fluiddicht gegen die Innenwandung 17 des Griffelementes 2 und insbesondere dessen Hartelement 13 eingeklemmt wird.

Wie erwähnt, ist um den Hüllschlauch 4 dieses Hartelement 13 des Griffelements 2 vollumfänglich angeordnet. Das Griffelement 2 abschließend ist das elastische Endelement 12 auf dem Hartelement 13 angeordnet, das den dichtenden Übergang zwischen dem Hartelement 13 des Griffelements 2 und dem Hüllschlauch 4 ausbildet und hier auf dem Hartelement 13 aufgespritzt ist. Auf diese Weise kann u.a. eine sichere Handhabung und ein Schutz vor dem Eindringen von Flüssigkeit oder Partikeln aus der Umgebung gewährleistet werden. Anstelle des Anspritzens ist es auch möglich, das elastische Endelement 12 als elastische Hülle über das Hartelement 13 und den Hüllschlauch 4 zu stülpen und es insbesondere derart auszubilden, dass es aktiv den Hüllschlauch 4 auf das Stützelement 8 presst.

Fig. 4 zeigt einen Querschnitt durch den Handgriff gemäß Fig. 1 entlang der Schnittlinie aus Fig. 2 in dessen proximalem Endbereich 10. Die dargestellten Bauteile entsprechen im Wesentlichen der Darstellung aus Fig. 3, jedoch ist hier das elastische Endelement 12 direkt auf dem Hüllschlauch 4 befestigt bzw. aufgespritzt zu sehen.

Fig. 5 zeigt eine Detailansicht des Arretierungsbereiches des Hüllschlauches, wie er in Fig. 2 gekennzeichnet ist. Um die Befestigung des Hüllschlauchs 4 mit seinem distalen Ende 18 an der Hülse 8 zu optimieren, kann über den Hüllschlauch 4 in Richtung der Klemmfläche 20 ein Klemmelement, insbesondere ein Klemmring 16, übergeschoben werden. Dieser Klemmring 16 weist ebenfalls eine Klemmfläche 20' auf, die eine komplementär zur Abschrägung der Klemmfläche 20 der Hülse 8 ausgebildete Abschrägung besitzt.

Zur zusätzlichen Abdichtung ist bei dieser Ausführungsform hinter dem Klemmring 16 ein Dichtelement, insbesondere ein 0-Ring 22, eingefügt. Für diesen 0-Ring 22 befindet sich im Griffelement 2 eine Ausnehmung 21, um einen gesicherten Halt des 0-Rings 22 zu ermöglichen. Diese Ausnehmung 21 ist im Hartelement 13 des Griffelements 2 ausgebildet.

Bei diesem Ausführungsbeispiel ist auch deutlich zu sehen, dass das elastische Endelement 12 direkt auf das Hartelement 13 des Griffelements 2 und in Richtung des proximalen Endes 10 (siehe Fig. 2) direkt auf dem Hüllschlauch 4 aufgebracht, vorzugsweise aufgespritzt ist. Dies führt zu einem doppelt abgedichteten Übergang zum Hüllschlauch 4. Dieser Übergang und seine Dichtwirkung werden bei einer Temperaturerhöhung (erhitzen) und anschließender Temperaturverminderung (abkühlen) noch verstärkt, da das elastische Endelement 12 mit seinen speziellen thermoplastischen Eigenschaften bei einer Erhöhung und anschließenden Verminderung der Temperatur nachschrumpft und sich auf dem Hüllschlauch 4 und dem Hartelement 13 des Griffelements 2 zusammenzieht; diese also noch mehr anpresst. Für das chirurgische Instrument 30 kann so eine sichere und optimale Abdichtung gegen Feuchtigkeit oder Partikel aus der Umgebung, insbesondere während des Reinigungs- bzw. Sterilisationsvorgangs oder eines chirurgischen Einsatzes in jeglichen Chirurgiegebieten, gewährleistet werden.

### Bezugszeichenliste

- 1 :: Handgriff
- 2 :: Griffelement
- 3 :: Sondenkopf
- 4 :: Hüllschlauch
- 5 :: Sondenspitze
- 6 :: Anschlussbereich
- 7 :: Versorgungseinheit
- 8 :: Stützelement bzw. Hülse
- 9 :: Schlauch
- 10 :: Proximales Ende
- 11 :: Distales Ende
- 12 :: Elastisches Endelement
- 13 :: Hartelement
- 14 :: Proximales Ende
- 15 :: Innenraum
- 16 :: Klemmelement bzw. Klemmring
- 17 :: Innenwandung
- 18 :: Distales Ende
- 19 :: Ausnehmung
- 20 :: Klemmfläche
- 22 :: Dichtelement bzw. O-Ring
- 23 :: Ausnehmung
- 30 :: Chirurgisches Instrument

## Patentansprüche

1. Handgriff für ein Chirurgieinstrument, insbesondere Kryochirurgieinstrument, mit einem Griffelement (2) und
wenigstens einem Hüllschlauch (4), der in einen am proximalen Ende (10) des Griffelements (2) ausgebildeten Anschlussbereich (6) in das Griffelement (2) eingeschoben und fluiddicht mit diesem verbunden ist, wobei das Griffelement (2) im Anschlussbereich (6) wenigstens teilweise aus einem elastischen Endelement (12) ausgebildet ist, und wobei im Innenraum des Hüllschlauches (4) koaxial wenigstens ein Stützelement (8) derart ausgebildet und angeordnet ist, dass der Hüllschlauch (4) im Anschlussbereich (6) zwischen dem Griffelement (2) und dem Stützelement (8) eingeklemmt ist, wobei das Stützelement (8) mit einem proximalen Ende aus dem Anschlussbereich (6) des Griffelements (2) und dem elastischen Endelement (12) hervorsteht, so dass der Hüllschlauch (4) über den Anschlussbereich (6) und das elastische Endelement (12) des Griffelements (2) hinaus gestützt wird.

2. Handgriff nach Anspruch 1, wobei das elastische Endelement (12) des Griffelements (2) aus einem Kunststoffmaterial und insbesondere aus einem thermoplastischen Elastomer ausgebildet ist, das sich bei einer Temperaturerhöhung zusammenzieht.

3. Handgriff nach einem der vorhergehenden Ansprüche, wobei das elastische Endelement (12) des Griffelements (2) um den Hüllschlauch (4) angespritzt oder dergleichen befestigt ist.

4. Handgriff nach einem der vorhergehenden Ansprüche, wobei das Stützelement (8) als eine Hülse ausgebildet ist.

5. Handgriff nach einem der vorhergehenden Ansprüche, wobei das Stützelement (8) mit einem proximalen Ende (14) aus dem Anschlussbereich (6) des Griffelements (2) um eine Länge (a) zwischen 1 mm bis 10 mm, insbesondere 5 mm, übersteht.

6. Handgriff nach einem der vorhergehenden Ansprüche, **gekennzeichnet durch** wenigstens ein Klemmelement (16), das den Hüllschlauch (4) insbesondere mit einem distalen Ende (18) auf dem Stützelement (8) einklemmt.

7. Handgriff nach Anspruch 6, wobei das Klemmelement (16) als ein Klemmring ausgebildet ist, der koaxial über den Hüllschlauch (4) in Richtung einer Klemmfläche (20) am Stützelement (8) derart aufschiebbar ist, dass der Hüllschlauch (4) zwischen Klemmfläche (20) und Klemmring (16) eingeklemmt ist.

8. Handgriff nach einem der vorhergehenden Ansprüche, wobei wenigstens ein Dichtelement (22), insbesondere ein O-Ring, dichtend zwischen dem Griffelement (2) und dem Hüllschlauch (4) und/oder zwischen dem Hüllschlauch (4) und dem Stützelement (8) angeordnet ist.

## Claims

1. Handle for a surgical instrument, in particular cryosurgery instrument, having at least one grip element (2) and
at least one cladding hose (4) which is inserted into the grip element (2) in a connecting region (6) formed at the proximal end (10) of the grip element (2) and is connected fluid-tightly therewith, wherein the grip element (2) in the connecting region (6) is formed at least partially from an elastic end element (12),
and wherein at least one supporting element (8) is configured and arranged coaxially in the interior of the cladding hose (4) such that the cladding hose (4) is clamped in the connecting region (6) between the grip element (2) and the supporting element (8), wherein a proximal end of the support element (8) protrudes from the connecting region (6) of the grip element (2) and the elastic end element (12) so that the cladding hose (4) is supported beyond the connecting region (6) and the elastic end element (12) of the grip element (2).

2. Handle according to claim 1, wherein the elastic end element (12) of the grip element (2) is made of a plastic material, in particular a thermoplastic elastomer, which contracts under a temperature increase.

3. Handle according to one of the preceding claims, wherein the elastic element (12) of the grip element (2) is moulded around the cladding hose (4) or similarly attached.

4. Handle according to any of the preceding claims, wherein the supporting element (8) is configured as a sleeve.

5. Handle according to any of the preceding claims, wherein a proximal end (14) of the supporting element (8) protrudes from the connecting region (6) of the grip element (2) by a length (a) between 1 mm and 10 mm, in particular 5 mm.

6. Handle according to any of the preceding claims, **characterised by** at least one clamping element (16) which clamps the cladding hose (4), in particular by a distal end (18), onto the supporting element (8).

7. Handle according to claim 6, wherein the clamping element (16) is formed as a clamping ring which can be pushed coaxially over the cladding hose (4) in the direction of a clamping face (20) on the supporting element (8) such that the cladding hose (4) is clamped between the clamping face (20) and the clamping ring (16).

8. Handle according to any of the preceding claims, wherein at least one sealing element (22), in particular an O-ring, is sealingly arranged between the grip element (2) and the cladding hose (4), and/or between the cladding hose (4) and the supporting element (8).

## Revendications

1. Poignée pour un instrument chirurgical, notamment un instrument de cryochirurgie, comprenant un élément formant poignée (2) et au moins un tuyau de gainage (4) qui est inséré dans l'élément formant poignée (2), dans une zone de raccordement (6) formée à l'extrémité proximale (10) de l'élément formant poignée (2), et est relié de manière étanche aux fluides à celui-ci, l'élément formant poignée (2) étant constitué, dans la zone de raccordement (6), au moins en partie d'un élément d'extrémité (12) élastique, et au moins un élément de support (8) étant réalisé et disposé de façon coaxiale à l'intérieur du tuyau de gainage (4), de manière à ce que, dans la zone de raccordement (6), le tuyau de gainage (4) soit serré entre l'élément formant poignée (2) et l'élément de support (8), l'élément de support (8) dépassant avec une extrémité proximale de la zone de raccordement (6) de l'élément formant poignée (2) et de l'élément d'extrémité (12) élastique, de sorte que le tuyau de gainage (4) est soutenu au-delà de la zone de raccordement (6) et de l'élément d'extrémité (12) élastique de l'élément formant poignée (2).

2. Poignée selon la revendication 1, dans laquelle l'élément d'extrémité (12) élastique de l'élément formant poignée (2) est réalisé à partir d'une matière plastique et notamment à partir d'un élastomère thermoplastique qui se contracte en cas d'augmentation de la température.

3. Poignée selon l'une des revendications précédentes, dans laquelle l'élément d'extrémité (12) élastique de l'élément formant poignée (2) est moulé par injection autour du tuyau de gainage (4) ou est fixé d'une manière similaire.

4. Poignée selon l'une des revendications précédentes, dans laquelle l'élément de support (8) est réalisé sous forme de manchon.

5. Poignée selon l'une des revendications précédentes, dans laquelle l'élément de support (8) dépasse avec une extrémité proximale (14) de la zone de raccordement (6) de l'élément formant poignée (2), d'une longueur (a) qui est comprise entre 1 mm et 10 mm et est notamment de 5 mm.

6. Poignée selon l'une des revendications précédentes, **caractérisée en ce qu'**elle comprend au moins un élément de serrage (16) qui serre le tuyau de gainage (4) notamment avec une extrémité distale (18) sur l'élément de support (8).

7. Poignée selon la revendication 6, dans laquelle l'élément de serrage (16) est réalisé sous forme de bague de serrage qui peut être glissée de façon coaxiale sur le tuyau de gainage (4), en direction d'une surface de serrage (20) de l'élément de support (8), de manière à ce que le tuyau de gainage (4) soit serré entre la surface de serrage (20) et la bague de serrage (16).

8. Poignée selon l'une des revendications précédentes, dans laquelle au moins un élément d'étanchéité (22), en particulier un joint torique, est disposé de façon à établir l'étanchéité entre l'élément formant poignée (2) et le tuyau de gainage (4) et/ou entre le tuyau de gainage (4) et l'élément de support (8).
